# EUROPEAN PATENT SPECIFICATION

(11) **EP 3 114 481 B1**
(45) Date of publication and mention of the grant of the patent: **06.12.2017**
(21) Application number: 15710227.8
(22) Date of filing: 04.03.2015
(51) Int. Cl.: G01N 33/53

(54) **ASSAY**
ASSAY
ESSAI

(30) Priority: 04.03.2014 GB 201403815
(43) Date of publication of application: 11.01.2017
(73) Proprietor: Mologic Limited, Thurleigh, Bedfordshire MK44 2YP (GB)
(72) Inventor: DAVIS, Paul, Sharnbrook Bedfordshire MK44 1PY (GB); HEMMINGTON, Sandra, Bedford Bedfordshire MK40 4AG (GB)
(74) Representative: Boult Wade Tennant
(86) International application number: PCT/GB2015/050624
(87) International publication number: WO 2015/132588

(56) References cited:
- WO-A1-2007/069940

### SEQUENCE LISTING

<110> Mologic Limited
<120> ASSAY
<130> MPS/P139070WO00
<150> GB1403815.2
   <151> 2014-03-04
<160> 3
<170> PatentIn version 3.5
<210> 1
   <211> 164
   <212> PRT
   <213> Artificial Sequence
<220>
   <223> synthetic polypeptide
<400> 1
   His His His His His His His Asn Glu Leu Ser His Phe Leu Glu Gly
   1               5                   10                  15
   Ser Ser Ser Ser Lys Glu Pro Leu Arg Pro Arg Cys Arg Pro Ile Asn
               20                  25                  30
   Ala Thr Leu Ala Val Glu Lys Glu Gly Cys Pro Val Cys Ile Thr Val
           35                  40                  45
   Asn Thr Thr Ile Cys Ala Gly Tyr Cys Pro Thr Met Thr Arg Val Leu
       50                  55                  60
   Gln Gly Val Leu Pro Ala Leu Pro Gln Val Val Cys Asn Tyr Arg Asp
   65                  70                  75                  80
   Val Arg Phe Glu Ser Ile Arg Leu Pro Gly Cys Pro Arg Gly Val Asn
                   85                  90                  95
   Pro Val Val Ser Tyr Ala Val Ala Leu Ser Cys Gln Cys Ala Leu Cys
               100                 105                 110
   Arg Arg Ser Thr Thr Asp Cys Gly Gly Pro Lys Asp His Pro Leu Thr
           115                 120                 125
   Cys Asp Asp Pro Arg Phe Gln Asp Ser Ser Ser Ser Lys Ala Pro Pro
       130                 135                 140
   Pro Ser Leu Pro Ser Pro Ser Arg Leu Pro Gly Pro Ser Asp Thr Pro
   145                 150                 155                 160
   Ile Leu Pro Gln
<210> 2
   <211> 157
   <212> PRT
   <213> Artificial Sequence
<220>
   <223> synthetic polypeptide
<400> 2
   Asn Glu Leu Ser His Phe Leu Glu Gly Ser Ser Ser Ser Lys Glu Pro
   1               5                   10                  15
   Leu Arg Pro Arg Cys Arg Pro Ile Asn Ala Thr Leu Ala Val Glu Lys
               20                  25                  30
   Glu Gly Cys Pro Val Cys Ile Thr Val Asn Thr Thr Ile Cys Ala Gly
           35                  40                  45
   Tyr Cys Pro Thr Met Thr Arg Val Leu Gln Gly Val Leu Pro Ala Leu
       50                  55                  60
   Pro Gln Val Val Cys Asn Tyr Arg Asp Val Arg Phe Glu Ser Ile Arg
   65                  70                  75                  80
   Leu Pro Gly Cys Pro Arg Gly Val Asn Pro Val Val Ser Tyr Ala Val
                   85                  90                  95
   Ala Leu Ser Cys Gln Cys Ala Leu Cys Arg Arg Ser Thr Thr Asp Cys
               100                 105                 110
   Gly Gly Pro Lys Asp His Pro Leu Thr Cys Asp Asp Pro Arg Phe Gln
           115                 120                 125
   Asp Ser Ser Ser Ser Lys Ala Pro Pro Pro Ser Leu Pro Ser Pro Ser
       130                 135                 140
   Arg Leu Pro Gly Pro Ser Asp Thr Pro Ile Leu Pro Gln
   145                 150                 155
<210> 3
   <211> 22
   <212> PRT
   <213> Artificial Sequence
<220>
   <223> synthetic polypeptide
<220>
   <221> SITE
   <222> (9)..(9)
   <223> Xaa = a linker having a length "m" which may be from about 3 to about 100 residues in length and may be, for example, an amino acid sequence, polyethylene glycol (PEG), VA or beta-alanine
<220>
   <221> SITE
   <222> (16)..(16)
   <223> Xaa = a linker having a length "n" which may be from about 3 to about 100 residues in length and may be, for example, an amino acid sequence, polyethylene glycol (PEG), VA or beta-alanine
<400> 3
   Asn Glu Leu Ser His Phe Leu Glu Xaa Ser Ile Arg Leu Pro Gly Xaa
   1               5                   10                  15
   Pro Val Val Ser Tyr Ala
               20

## Description

The present invention relates to a method and a kit for determining the presence of an analyte in a liquid sample.

Immunoassays for determining the presence of an analyte in a liquid sample are well known. A common type of immunoassay is a sandwich immunoassay. In such an assay, sample is added to a test plate coated with a capture antibody. Analyte present in the sample binds the capture antibody and is retained on the plate. The plate is washed to remove unbound analyte and a detecting antibody is then added which binds the analyte which is bound to the capture antibody thereby forming an analyte sandwich between the two antibodies. The analyte sandwich can be detected, either by virtue of a label attached to the detecting antibody or by the addition of a further, labelled antibody which binds to the detecting antibody. The amount of detected label is directly proportional to the concentration of the analyte in the sample.

An alternative approach is a competitive inhibition assay. Again, a capture antibody is provided on a test plate. Sample and a labelled version of the analyte of interest are added to the test plate. Labelled analyte competes with analyte present in the sample for binding to the capture antibody. The plate is washed and the amount of captured labelled analyte present is determined. The amount of labelled analyte detected is typically inversely proportional to the concentration of analyte in the sample.

Known assays suffer from various problems. For example, with certain assay formats steric hindrance may compromise the formation of an immuno-complex between an antibody and an analyte, for example when such interactions occur at a surface.

WO2007/069940 A1 discloses methods and devices for determining the amount of an analyte in a sample.

The present invention is defined in the claims. In a first aspect, the present invention provides methods of determining the presence of an analyte present in a liquid sample. The invention provides a method of determining the presence of an analyte present in a liquid sample, the method comprising:
a) contacting the liquid sample with a predetermined amount of an analogue of the analyte and a first binding moiety, wherein the analogue comprises an amino acid sequence which has been recombinantly expressed or synthesized *in vitro* to include a binding region composed of amino acids that can be bound by a second binding moiety, such that the analogue incorporates only one of said binding region, wherein the binding region is not present in the analyte or is present in the analyte but not accessible to the second binding moiety; and
   the first binding moiety is capable of binding each of the analyte and the analogue independently, such that a mixture is formed comprising an analogue-first binding moiety complex, or, when analyte is present in the sample, either (i) an analogue-first binding moiety complex and an analyte-first binding moiety complex, or (ii) an analyte-first binding moiety complex and no analogue-first binding moiety complex;
b) contacting the mixture with the second binding moiety, wherein the second binding moiety is an antibody capable of binding the analogue-first binding moiety complex but not the analyte-first binding moiety complex, and is immobilised or immobilisable on a solid phase;
c) determining the level of a signal indicative of the presence of the analogue-first binding moiety complex bound to the second binding moiety;
wherein if the level of the signal determined in step c) is lower than the level of a maximum signal determined when no analyte is present, then analyte is present in the sample.

In step (a) above, the first binding moiety may be capable of binding both the analogue and analyte simultaneously.

The invention further provides a method of determining the presence of an analyte present in a liquid sample, the method comprising:
a) contacting the liquid sample with a predetermined amount of an analogue of the analyte and a first binding moiety, wherein the analogue comprises an amino acid sequence which has been recombinantly expressed or synthesized *in vitro* to include a binding region composed of amino acids that can be bound by a second binding moiety, such that the analogue incorporates only one of said binding region, wherein the binding region is not present in the analyte or is present in the analyte but not accessible to the second binding moiety; and
   the first binding moiety is capable of binding each of the analyte and the analogue independently but not both the analogue and analyte simultaneously, such that a mixture is formed comprising an analogue-first binding moiety complex, or, when analyte is present in the sample, either (i) an analogue-first binding moiety complex and an analyte-first binding moiety complex, or (ii) an analyte-first binding moiety complex and no analogue-first binding moiety complex;
b) contacting the mixture with the second binding moiety, wherein the second binding moiety is an antibody capable of binding the analogue-first binding moiety complex but not the analyte-first binding moiety complex, and is immobilised or immobilisable on a solid phase;
c) determining the level of a signal indicative of the presence of the analogue-first binding moiety complex bound to the second binding moiety;
wherein if the level of the signal determined in step c) is lower than the level of a maximum signal determined when no analyte is present, then analyte is present in the sample.

The present invention provides a number of advantages over prior art assay formats, which include formation of a first antibody-target complex in solution phase; operation of a competitive immunoassay format in solution phase, followed by surface capture and separation of bound from free label that involves fewer washing steps compared with prior art assay formats. The methods of the invention may enable the presence of an analyte in a liquid sample to be determined more quickly than by known methods, for example, the RIA assay and/or Immulite assay. Preferably, the methods of the invention enable the presence of an analyte in a liquid sample to be determined in under 24 hours, under 12 hours or under 4 hours.

Once the liquid sample is contacted with the analogue and the first binding moiety, the analogue, first binding moiety and, where present the analyte, are free to diffuse and interact with one another in solution according to the well-known principles of Brownian motion. When there is no analyte in the sample, the first binding moiety can bind only to the analogue, and a mixture comprising analogue-first binding moiety complex is formed. This mixture is contacted with the second binding moiety. The second binding moiety binds the analogue (even when it is in complex with the first binding moiety), and therefore analogue-first binding moiety complex is captured on the second binding moiety, which is preferably provided in excess. The second binding moiety may be immobilised, for example, on a surface such as the well of a microtitre plate. Alternatively, the second binding moiety may be immobilisable, such as by means of an immobilised binding moiety, such that it becomes immobilised on a surface either before or after it has bound the analogue-first binding moiety complex. In this case, the method may comprise the additional step of contacting the second binding moiety with a surface in order to immobilise the second binding moiety on the surface. Any first binding moiety that has not been captured by the second binding moiety may be separated from the captured analogue-first binding moiety complex before step (c) is carried out. This avoids the subsequent detection of any first binding moiety that has not been captured by the second binding moiety. Any suitable separation method may be used, for example, washing with buffer, washing with air, magnetic separation, filtration or immunochromatography. The level of a signal indicative of the presence of the captured analogue-first binding moiety complex is then determined.

The signal is preferably derived (either directly or indirectly) from a label, which is either directly or indirectly attached to or associated with the first-binding moiety. Irrespective of how the signal is produced and how the label is associated with the first binding moiety, the level of signal will be proportional to the amount of analogue-first binding moiety captured by the second binding moiety.

The first binding moiety may itself comprise a label. Alternatively, the first binding moiety may not comprise a label, and the method may further comprise contacting the first binding moiety with a third binding moiety, wherein the third binding moiety binds the first binding moiety (either directly or indirectly) and comprises a label. Thus, in such an embodiment, the labelled third binding moiety provides the signal indicative of the presence of the captured analogue-first binding moiety complex. The third binding moiety may be contacted with the first binding moiety at any stage of the method before the level of signal is determined. For example, the third binding moiety could be contacted with the first binding moiety before the liquid sample is contacted with the first binding moiety, after this step but before the mixture is contacted with the second binding moiety, or after the mixture is contacted with the second binding moiety but before the level of signal is determined. The third binding moiety may be contacted with the first binding moiety when the first binding moiety has formed a complex with the analogue and the complex has been captured by the second binding moiety. One or more intermediate binding moieties could be used to link the third binding moiety or label to the first binding moiety. The signal may be produced directly or indirectly by the label. For example, the label may bring about the production of the signal by reaction with a reagent. In this case, the method may further include the step of adding a suitable reagent in order to produce the signal. Suitable labels are discussed in detail below.

The level of signal detected when no analyte is present in the sample is the maximum signal for the test. This is because there is no analyte to compete with the analogue for binding to the first binding moiety and thus the maximum amount of analogue-first binding moiety-complex is formed and captured by the second binding moiety. As such, a maximum signal can be obtained by carrying out the method of the invention on a sample known not to contain the analyte. This maximum signal can be compared with tests carried out under the same conditions (e.g. concentration of first binding moiety, analogue and second binding moiety) on samples with unknown analyte content, in order to determine whether, and how much, analyte is present.

The first binding moiety is capable of binding to each of the analyte and the analogue independently but preferably not both the analyte and the analogue simultaneously. Preferably, the affinity of the first binding moiety for the analyte is substantially the same as the affinity of the first binding moiety for the analogue. The sample and the analogue may be contacted with the first binding moiety simultaneously. In this case, when analyte is present in the sample, there is direct competition between the analogue and the analyte for binding to the first binding moiety. Alternatively, the sample may be contacted with the first binding moiety before either the sample or the first binding moiety is contacted with the analogue. In this case, when analyte is present in the sample it binds to a proportion of the available first binding moiety. The analogue can then only bind to first binding moiety that is not occupied by the analyte (if indeed any first binding moiety is not occupied by analyte). In both embodiments, the analyte is said to compete with the analogue for binding to the first binding moiety, and analyte-first binding moiety complex is formed in an amount proportional to the concentration of analyte in the sample. Consequently less analogue-first binding moiety complex is formed than when no analyte is present in the sample. If the analyte is present in a very high concentration, it may completely out-compete the analogue for binding to the first binding moiety, such that no analogue-first binding moiety is formed. Thus, a mixture is formed which comprises either (i) an analogue-first binding moiety complex and an analyte-first binding moiety complex, or (ii) an analyte-first binding moiety complex only.

The mixture is contacted with the second binding moiety (which may or may not be immobilised on a surface). As there is less analogue-first binding moiety complex present in the mixture (or no analogue-first binding moiety complex at all) than when analyte is not present in the sample, the amount of analogue-first binding moiety complex captured by the second binding moiety is also less than the amount captured when no analyte is present in the sample. The second binding moiety does not bind the analyte-first binding moiety complex, and therefore, this complex is not captured by the second binding moiety. The second binding moiety does not have a binding affinity for the analyte. This means that the maximum amount of second binding moiety is available to capture analogue-first binding moiety complex, because the second binding moiety only has a binding affinity for the analogue.

After the second binding moiety has been contacted with the mixture, the level of a signal indicative of the presence of the analogue-first binding moiety complex bound to the second binding moiety is determined as described above. Where the second binding moiety is not immobilised on a surface when it binds the analogue-first binding moiety complex, the method preferably includes the step of contacting the second binding moiety with a surface such that it becomes immobilised before the level of signal is determined.

When analyte is present in the sample, the level of signal determined in step (c) will be lower than the level of signal determined when there is no analyte present in the sample, because less analogue-first binding moiety complex is formed and captured by the second binding moiety. In other words, the signal detected is inversely proportional to the concentration of analyte in the sample.

The method of the invention can also be used to determine the amount or concentration of analyte present in the sample. To achieve this, the level of signal determined in step (c) is typically compared to calibration data. Calibration data can be obtained by conducting controls in which the level of signal produced for a series of known analyte concentrations is determined. The concentration of analogue, first binding moiety and second binding moiety should be kept constant for each analyte concentration tested. Similarly, the concentration of each of these used in a test should be the same as the concentration used to produce the calibration data with which the test result is to be compared. Thus, the method of the invention may comprise determining the amount or concentration of the analyte present in the sample by comparing the level of signal determined in step (c) with calibration data.

As the level of signal detected is inversely proportional to the concentration of analyte, the higher the level of signal, the lower the concentration of analyte in the sample and conversely, the lower the level of signal, the higher the concentration of analyte in the sample. This inverse relationship is particularly useful when the analyte is present at very low levels. This is because the level of signal that is detected in these circumstances is high and thus easier to detect than in conventional assays where the signal level is proportional to the concentration of analyte (and thus a very low signal is produced when the analyte is present in a low concentration). Therefore, the method of the present invention has the advantage of being highly sensitive to low levels of analyte.

The analyte can be any entity that is capable of being bound by the first binding moiety of the present invention. For example, the analyte may be or may comprise a peptide, a polypeptide, a protein, nucleic acid, a polynucleotide, a carbohydrate, an etiological agent, a hormone, a vitamin, a steroid, a drug (for example a drug of abuse or DOA), an infectious agent or an entity indicative of an infected state, a microorganism, a bacterium, a virus, a toxin, an organic compound, a pollutant, a pesticide, a biomarker for a disease, a biomarker for pregnancy or metabolites of or antibodies to any of the above. The term analyte also includes any antigenic substance, haptens, antibodies, macromolecules, and combinations thereof. Preferably, the analyte comprises an epitope recognised by an antibody. In one embodiment, the analyte is human chorionic gonadotropin (hCG) or a fragment or portion thereof, including intact dimeric hCG, free alpha subunit, free beta subunit, and clipped, cleaved or truncated forms of intact dimeric hCG, free alpha subunit, free beta subunit, including glycosylation variants and in particular may include free-beta core.

Human chorionic gonadotropin (hCG) is an important biomarker in pregnancy and oncology, where it is routinely detected and quantified by specific immunoassays. Essential in pregnancy and detectable within a few days of fertilization, hCG has become one of the most frequently assayed hormones, and simple, one-step, antibody based measurements of hCG are now standard in pregnancy testing. Furthermore, the role of hCG assays in the diagnosis and management of malignant trophoblastic disease (choriocarcinoma) is one of the great success stories of oncology. It is an ideal tumour marker, always present when choriocarcinoma cells exist, and in quantities directly related to the number of those cells. The correct use of hCG assays in combination with appropriate therapy has led to a survival rate approaching 100% in this otherwise aggressive cancer (Gregor, CR et al., J 2011, 'Antibody Recognition of a Human Chorionic Gonadotropin Epitope (hCG beta66-80) Depends on Local Structure Retained in the Free Peptide', Journal of Biological Chemistry, vol 286, no. 28, pp. 25016-25026).

The analogue of the analyte is related to the analyte in that the first binding moiety is capable of binding both the analogue and the analyte, preferably with substantially equal affinity. Thus, the analogue and the analyte may share a degree of structural, sequence, chemical or immunological similarity, and may comprise a binding epitope. For example, if the analyte comprises an amino acid or nucleic acid sequence, the analogue may share a degree of sequence identity with the sequence of the analyte. In some embodiments, the analogue comprises a sequence that is identical to either the full analyte sequence or a portion of the analyte sequence. In other embodiments, the analogue comprises a sequence that is at least 75%, at least 80%, at least 85%, at least 90%, at least 95%, at least 96%, at least 97%, at least 98%, at least 99% identical to the full analyte sequence or a portion of the analyte sequence. The portion of the analyte sequence that is identical to a portion of the analogue sequence may be from about 5 to about 1000 residues in length. For example, the portion may be about 5 to about 10, about 10 to about 15, about 15 to about 20, about 20 to about 30, about 30 to about 50, about 50 to about 70, about 70 to about 100, about 100 to about 150, about 150 to about 250, about 250 to about 500, about 500 to about 750, or about 750 to about 1000 residues in length.

The analogue-first binding moiety complex can be bound by the second binding moiety. Preferably, the analogue itself is capable of being bound by the second binding moiety. However, it may be that the second binding moiety only binds the complex of the analogue and the first binding moiety. In contrast, the second binding moiety cannot bind the analyte-first binding moiety complex. Therefore, as defined in the claims, the analogue comprises an amino acid sequence which has been recombinantly expressed or synthesized *in vitro* to include a binding region composed of amino acids that can be bound by the second binding moiety that is not present in the analyte, or that is present but is not capable of being bound by the second binding moiety e.g. it is in a position in the analyte that is not accessible to the second binding moiety. For example the analogue may consist exclusively of an amino acid sequence. The analogue comprises an amino acid sequence and the binding region recognised by the second binding moiety may be at the N-terminus, at the C-terminus or at any other position in the amino acid sequence of the analogue.

The amino acid sequence of the analogue is preferably expressed recombinantly to include an amino acid sequence (a binding region) that can be bound by the second binding moiety and that is not present in the analyte, or is present in the analyte but is not accessible to the second binding moiety. Alternatively, the analogue may be synthesized using standard peptide synthesis techniques. An advantage of expressing the analogue recombinantly or synthesising the analogue de novo to include an amino acid binding region recognised by the second binding moiety, is that only one of these binding regions is incorporated into each analogue molecule. If the binding region recognised by the second binding moiety is added to the analogue after the analogue has been synthesised, there is a possibility that multiple binding regions are incorporated into the analogue. The presence of multiple binding regions in the analogue may disrupt or inhibit the method of the invention. For example, multiple binding regions may prevent the analogue from competing with the analyte to bind the first binding moiety, or may prevent the analogue from binding to the second binding moiety when in complex with the first binding moiety.

N-terminal or C-terminal sequences may be of the type generally referred to as tags. Non-limiting examples include, in general, affinity tags, solubilisation tags, chromatography tags and epitope tags. Non-limiting examples of specific tags include FLAG-tag, MYC-tag, HA-tag, GST-tag, Strep-tag and a poly-histidine tag. The skilled person will be aware of many other general classes of tags and more specific examples of each class, and will be able to choose an appropriate second binding moiety accordingly. In some embodiments, the binding region may be a known epitope from an antigen or may be synthetic. If synthetic it may be designed specifically for the analogue in view of the analyte. An epitope for which a known antibody or antibody fragment is specific may form part of the analogue.

In one embodiment, the analyte is hCG and the analogue comprises an amino acid sequence that is 75%, 80%, 85%, 90%, 95%, 96%, 97%, 98%, 99% or 100% identical to the sequence of SEQ ID NO: 1, or SEQ ID NO: 2:
SEQ ID NO 1:
SEQ ID NO 2:

The underlined region of both sequences represents a unique epitope region, referred to herein as "ST068". The underlined region is bound by the second binding moiety and is not present in the analyte i.e. hCG.

The double underlined region of SEQ ID NOs 1 and 2, i.e. the sequence: SIRLPGCPRGVNP VVSYA, represents a binding domain known as beta-3 that is found in hCG. Thus, the analogue may comprise (and the first binding moiety may bind) a sequence comprising or consisting of the double underlined portion of SEQ ID NO: 1, i.e. residues 85 to 102 of SEQ ID NO: 1 or the double underlined portion of SEQ ID NO: 2, i.e. residues 78-95 of SEQ ID NO: 2. The analogue may comprise (and the second binding moiety may bind) a sequence comprising or consisting of residues 8-15 of SEQ ID NO: 1 or residues 1-8 of SEQ ID NO: 2, i.e. the sequence: NELSHFLE, which may be referred to as a TAG epitope. The analogue may comprise a sequence having at least 95%, at least 96%, at least 97%, at least 98%, at least 99% or 100% identity to the sequence: SIRLPGCPRGVNPVVSYA, and/or may comprise a sequence having at least 95%, at least 96%, at least 97%, at least 98%, at least 99% or 100% identity to the sequence: NELSHFLE. The analogue may include a polyhistidine tag e.g. residues 1-7 of SEQ ID NO: 1.

In other embodiments, in particular, those in which the analyte is hCG, the analogue comprises at least 157 amino acids, and may comprise at least amino acids 1-8 and 84-95 of SEQ ID NO: 2. Alternatively, the analogue may comprise at least 164 amino acids, and may comprise at least amino acids 1-15 and 95-102 of SEQ ID NO: 1. In one embodiment, the analogue has 50% identity to SEQ ID NO: 1, and comprises at least amino acids 1-15 and 71-83 of SEQ ID NO: 1.

In another embodiment, the analogue comprises NELSHFLEXₘSIRLPGZₙPVVSYA (SEQ ID NO: 3), wherein X is a linker having a length "m" and Z is a linker having a length "n". X and Z may be the same or may be different and may each independently be, for example, an amino acid sequence, polyethylene glycol (PEG), VA (see structure below) or beta-alanine.

VA linker:

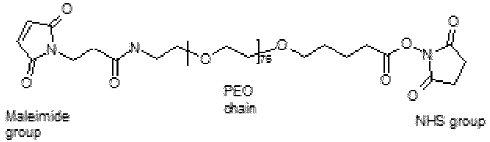

A function of linker X is to reduce steric interference between the TAG epitope and the hCG sequence. To this end a distance of at least 1.5nm (15 angstroms) should be sufficient. This is approximately equivalent to five amino acids or two AEEAc linkers (short alternatives to PEG) or an appropriate PEG linker (e.g. with two repeat units).

A function of linker Z is to encourage an antiparallel beta turn so that the two flanking sequences of hcG-beta3 align in a conformational epitope that can be bound by the first binding moiety. The affinity of the first binding moiety for this conformational epitope is important in the context of the assay and can be optimised by adjusting the properties of the linker, for example its length and amino acid content.

When X and/or Z is an amino acid sequence, "m" and "n" may each be from about 3 to about 100 residues in length, about 5 to about 90 residues in length, about 10 to about 80 residues in length, or about 20 to about 70 residues in length. For example, "m" and/ or "n" may be 3, 4 or 5 residues in length. In the embodiment where "m" and/or "n" is 3 residues in length, the sequence PPN may be used. The term amino acid used herein includes any molecule with an amino group and a carboxylic acid group including naturally occurring amino acids, non-naturally occurring amino acids and amino acids derivatives, such as, for example, AEEAc and amino-PEG-acids.

The amount of analogue required will depend on various factors. Typically, the analogue is provided in a low, limiting concentration. This may be around 4-8 picograms/ml but the concentration required will vary from batch to batch and an appropriate concentration can be determined by the skilled person.

The binding moiety (first, second and/or third) may be naturally derived or wholly or partially synthetic. The binding moiety and its target, (the analogue and the analyte are the targets of the first binding moiety, the analogue-first binding moiety complex is the target of the second binding moiety and the first binding moiety is the target of the third binding moiety where present) together form a pair of binding partners. The members of the pair have the property of binding specifically to each other. Examples of types of specific binding pairs are antigen-antibody, biotin-avidin, hormone-hormone receptor, complementary nucleotide sequences, complementary peptide sequences, receptor-ligand, enzyme cofactor-enzyme, enzyme inhibitor-enzyme, enzyme-substrate, carbohydrate-lectin, polymeric acid-base, dye-protein binder, peptide-specific protein binder (e.g., ribonuclease, S-peptide and ribonuclease S-protein), and the like. While the present invention is generally concerned with antigen-antibody type reactions and preferred binding moieties of the invention are antibodies or fragments thereof, other embodiments are also envisaged employing other types of binding moieties and binding pairs. As defined in the claims, the second binding moiety is an antibody.

The term "antibody" as used herein refers to immunoglobulin molecules and immunologically active portions of immunoglobulin molecules, i.e., molecules that contain an antigen binding site that specifically binds an antigen, whether natural or partly or wholly synthetic. The term also covers any polypeptide or protein having a binding domain which is, or is homologous to, an antibody binding domain. These can be derived from natural sources, or they may be partly or wholly synthetically produced. Examples of antibodies are the immunoglobulin isotypes (e.g., IgG, IgE, IgM, IgD and IgA) and their isotypic subclasses; fragments which comprise an antigen binding domain such as Fab, F(ab')₂, scFv, Fv, dAb, Fd; and diabodies. Antibodies may be polyclonal or monoclonal. A monoclonal antibody may be referred to herein as "mab".

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementary determining regions (CDRs), of an antibody to the constant regions, or constant regions plus framework regions, of a different immunoglobulin. See, for instance, EP-A-184187, GB 2188638A or EP-A-239400.

As antibodies can be made or modified in a number of ways, the term antibody should be construed as covering any specific binding member or substance having a binding domain with the required specificity. Thus, this term covers antibody fragments, derivatives, functional equivalents and homologues of antibodies, humanised antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023. A humanised antibody may be a modified antibody having the variable regions of a non-human, e.g. murine, antibody and the constant region of a human antibody. Methods for making humanised antibodies are described in, for example, US Patent No. 5225539.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341:544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., Science 242:423-426 (1988); Huston et al., PNAS USA 85:5879-5883 (1988)); (viii) bispecific single chain Fv dimers (PCT/US92/09965) and (ix) "diabodies", multivalent or multispecific fragments constructed by gene fusion (WO94/13804; P. Hollinger et al., Proc. Natl. Acad. Sci. USA 90: 6444-6448 (1993)).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Hollinger & Winter, Current Opinion Biotechnol. 4:446-449 (1993)), e.g. prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single chain "Janusins" described in Traunecker et al., EMBO Journal 10:3655-3659 (1991).

Bispecific diabodies, as opposed to bispecific whole antibodies, may also be useful because they can be readily constructed and expressed in *E. coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

An "antigen binding domain" is the part of an antibody which comprises the area which specifically binds to part or all of an antigen. Where an antigen is large, an antibody may only bind to a particular part of the antigen, which part is termed an epitope. An antigen binding domain may be provided by one or more antibody variable domains. An antigen binding domain may comprise an antibody light chain variable region (VL) and an antibody heavy chain variable region (VH).

"Specific" is generally used to refer to the situation in which the binding moiety will not show any significant binding to molecules other than its target(s), and, e.g., has less than about 30%, preferably 20%, 10%, 1%, 0.5%, 0.3%, 0.2% or 0.1% cross-reactivity with any other molecule. The term is also applicable where e.g. an antigen binding domain is specific for a particular epitope which is carried by a number of antigens, in which case, the binding moiety carrying the antigen binding domain will be able to bind to the various antigens carrying the epitope.

The first, second and third binding moieties do not necessarily have to be the same type of binding moiety, although they may be in some embodiments.

The first binding moiety may be an hCG β3-loop-specific monoclonal antibody, for example the antibody "8G5" (see Gregor et al., J Biol Chem. 2011 Jul 15;286(28):25016-26). The first binding moiety may be used at a concentration of about 1 ng/ml and may be an antibody-HRP conjugate.

The second binding moiety may be immobilised or immobilisable on a surface selected from the group comprising a microtiter plate, a nitrocellulose matrix, a glass fibre matrix, a particle, a membrane, a peg, a slide, a bibulous membrane, a surface of a microfluidic device, a chromatographic material. The particle may be, for example, a magnetic particle, a latex particle, a glass particle, a magnetically susceptible particle, or a colloidal metal particle

As mentioned, the signal is preferably derived (either directly or indirectly) from a label, which is either directly or indirectly attached to or associated with the first-binding moiety. "Label" refers to any substance which is capable of producing a signal that is detectable by visual or instrumental means. Various labels useful in the present invention include labels which produce signals through either chemical or physical means, such as being optically detectable. Such labels include enzymes and substrates, chromogens, catalysts, fluorescent compounds, chemiluminescent compounds, electroactive species, dye molecules, radioactive labels and particle labels. The first binding moiety itself may be inherently capable of producing a detectable signal. The label may be covalently attached to the first binding moiety. In particular, the label may be chosen from one that is optically detectable. When the label comprises an enzyme, the method may include a step of contacting the enzyme with a substrate in order to produce the signal. In one embodiment, the label is horseradish peroxidase (HRP).

The label may comprise a particle such as gold, silver, colloidal non-metallic particles such as selenium or tellurium, dyed or coloured particles such as a polymer particle incorporating a dye, or a dye sol. The dye may be of any suitable colour, for example blue. The dye may be fluorescent. Dye sols may be prepared from commercially- available hydrophobic dyestuffs such as Foron Blue SRP (Sandoz) and Resolin Blue BBLS (Bayer). Suitable polymer labels may be chosen from a range of synthetic polymers, such as polystyrene, polyvinyltoluene, polystyrene-acrylic acid and polyacrolein. The monomers used are normally water-insoluble, and are emulsified in aqueous surfactant so that monomer micelles are formed, which are then induced to polymerise by the addition of initiator to the emulsion. Substantially spherical polymer particles are produced. An ideal size range for such polymer particles is from about 0.05 to about 0.5um. According to an exemplary embodiment, the label is a blue polymeric particle or a gold particle.

As already mentioned, the second binding moiety may be immobilised on a surface or may not be immobilised at the beginning of the method. In the latter case, the second binding moiety may be contacted with the analogue-first binding moiety complex either before or after it has been contacted with a surface and immobilised.

The liquid sample can be derived from any source, such as an industrial, environmental, agricultural, or biological source. The sample may be derived from or consist of a physiological source including blood, serum, plasma, interstitial liquid, saliva, sputum, ocular lens liquid, sweat, urine, milk, ascites liquid, mucous, synovial liquid, peritoneal liquid, transdermal exudates, pharyngeal exudates, bronchoalveolar lavage, tracheal aspirations, cerebrospinal liquid, semen, cervical mucus, vaginal or urethral secretions and amniotic liquid. The liquid sample may be derived from a semi-solid or solid source by dilution, treatment or extraction into an aqueous liquid.

Under certain circumstances, the sample may be expected to contain a very high concentration of analyte. This may be the case, for example, if the sample has been obtained from a subject having an advanced disease state. In such circumstances, the sample may be diluted before it is contacted with the first binding moiety and analogue to bring the analyte concentration into an appropriate range for the assay.

In a second aspect, as defined in the claims, the invention provides test kits for detecting an analyte of interest. Thus, the invention provides a test kit for detecting an analyte of interest comprising
i) an analyte analogue;
ii) either (a) a labelled first binding moiety or (b) an unlabelled first binding moiety and a labelled third binding moiety; and
iii) an immobilisable second binding moiety
wherein the analogue comprises an amino acid sequence which has been recombinantly expressed or synthesized *in vitro* to include a binding region composed of amino acids that can be bound by the second binding moiety, such that the analogue incorporates only one of said binding region, wherein the binding region is not present in the analyte or is present in the analyte but not accessible to the second binding moiety; and
the first binding moiety is capable of binding each of the analogue and the analyte of interest independently, the second binding moiety is an antibody capable of binding a complex of the analogue and the first binding moiety but is not capable of binding the analyte or a complex of the first binding moiety and the analyte, and the third binding moiety is capable of binding the first binding moiety or the analogue-first binding moiety complex.

The first binding moiety may be capable of binding both the analogue and analyte simultaneously.

The invention further provides a test kit for detecting an analyte of interest comprising
i) an analyte analogue;
ii) either (a) a labelled first binding moiety or (b) an unlabelled first binding moiety and a labelled third binding moiety; and
iii) an immobilisable second binding moiety
wherein the analogue comprises an amino acid sequence which has been recombinantly expressed or synthesized *in vitro* to include a binding region composed of amino acids that can be bound by the second binding moiety, such that the analogue incorporates only one of said binding region, wherein the binding region is not present in the analyte or is present in the analyte but not accessible to the second binding moiety; and
the first binding moiety is capable of binding each of the analogue and the analyte of interest independently but not both the analogue and analyte simultaneously, the second binding moiety is an antibody capable of binding a complex of the analogue and the first binding moiety but is not capable of binding the analyte or a complex of the first binding moiety and the analyte, and the third binding moiety is capable of binding the first binding moiety or the analogue-first binding moiety complex.

The analyte, analogue, and first, second and third binding moieties may be as defined in relation to the first aspect of the invention.

Also described herein is a polypeptide that comprises or consists of an amino acid sequence that is 75%, 80%, 85%, 90%, 95%, 96%, 97%, 98%, 99% or 100% identical to the sequence of SEQ ID NO: 1, or SEQ ID NO: 2, or a polypeptide that comprises or consists of the amino acid sequence NELSHFLEXₙSIRLPGZₙPVVSYA (SEQ ID NO: 3), wherein X and Z are linkers having a length "m" and "n" respectively. X, Z, "m" and "n" are as defined in the first aspect of the invention.

The polypeptide may comprise a sequence having at least 95%, at least 96%, at least 97%, at least 98%, at least 99% or 100% identity to the sequence: SIRLPGCPRGVNPVVSYA, and/or may comprise a sequence having at least 95%, at least 96%, at least 97%, at least 98%, at least 99% or 100% identity to the sequence: NELSHFLE. The polypeptide may comprise at least 164 amino acids, and may comprise at least amino acids 1-15 and 95-102 of SEQ ID NO: 1. The polypeptide may comprise at least 157 amino acids, and may comprise at least amino acids 1-8 and 84-95 of SEQ ID NO: 2. In one embodiment, the polypeptide has 50% identity to SEQ ID NO: 1, and comprises at least amino acids 1-15 and 71-83 of SEQ ID 1.

The polypeptides described herein may be provided in isolated or recombinant form, and may be fused to other moieties. The polypeptides may be provided in substantially pure form, that is to say free, to a substantial extent, from other proteins. Thus, a polypeptide may be provided in a composition in which it is the predominant component present (i.e. it is present at a level of at least 50%; preferably at least 75%, at least 90%, or at least 95%; when determined on a weight/weight basis excluding solvents or carriers).

Also described herein is a nucleic acid encoding a polypeptide as described herein.

As used herein with respect to nucleic acid molecules, isolated or recombinant means any of a) amplified *in vitro* by, for example, polymerase chain reaction (PCR), b) recombinantly produced by cloning, c) purified by, for example, gel separation, or d) synthesised, such as by chemical synthesis.

The nucleic acid, for example DNA and RNA, may be synthesised using methods known in the art, such as using conventional chemical approaches or polymerase chain reaction (PCR) amplification or other methods of amplification.

Also described herein is an expression vector comprising nucleic acid encoding a polypeptide as described herein.

A variety of host-expression vector systems may be utilised to express a polypeptide as described herein. Such host-expression systems represent vehicles by which the coding sequences of interest may be produced and subsequently purified, but also represent cells which may, when transformed, transduced or transfected with the appropriate nucleotide coding sequences, express the polypeptide as described herein *in situ.* These include but are not limited to microorganisms such as bacteria (e.g., *E. coli, B. subtilis*) transformed with recombinant bacteriophage DNA, plasmid DNA or cosmid DNA expression vectors containing polypeptide coding sequences; yeast (e.g., *Saccharomyces, Pichia*) transformed with recombinant yeast expression vectors containing polypeptide coding sequences; insect cell systems infected with recombinant virus expression vectors (e.g., baculovirus) containing the polypeptide coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing polypeptide coding sequences; or mammalian cell systems (e.g., COS, CHO, BHK, 293, 3T3 cells) harbouring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the polypeptide being expressed. For example, when a large quantity of such a polypeptide is to be produced, vectors which direct the expression of high levels of fusion protein products that are readily purified may be desirable. Such vectors include, but are not limited, to the *E. coli* expression vector pUR278 (Ruther et al., 1983, EM80 J. 2:1791); pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix comprising glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The polypeptide coding sequence may be cloned individually into non-essential regions (for example, the polyhedrin gene) of the virus and placed under control of an AcNPV promoter (for example, the polyhedrin promoter). In mammalian host cells, a number of viral-based expression systems (e.g., an adenovirus expression system) may be utilised.

As discussed above, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein.

For long-term, high-yield production of polypeptides of the invention, stable expression is preferred. For example, cells lines that stably express the polypeptide can be produced by transfecting the cells with an expression vector comprising the nucleotide sequence of the polypeptide and the nucleotide sequence of a selectable (e.g., neomycin or hygromycin), and selecting for expression of the selectable marker. Such engineered cell lines may be particularly useful in screening and evaluation of compounds that interact directly or indirectly with the polypeptide.

The expression levels of the polypeptide of the invention can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing the polypeptide is amplifiable, increase in the level of inhibitor present in culture of host cell will increase the number of copies of the marker gene. Since the amplified region is associated with the polypeptide gene, production of the polypeptide will also increase (Crouse et al., 1983, Mol. Cell. Biol. 3:257).

Once the polypeptide has been expressed recombinantly, it may be purified by any method known in the art for purification of a polypeptide, for example, by chromatography (e.g., ion exchange chromatography, affinity chromatography such as with protein A, poly-histidine or specific antigen, and sizing column chromatography), centrifugation, differential solubility, or by any other standard technique for the purification of proteins.

Alternatively, any fusion protein may be readily purified by utilising an antibody specific for the fusion protein being expressed. For example, a system described by Janknecht *et al.* allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

In one embodiment of the invention, in a first reaction phase, a labelled antibody is incubated in the presence of a limiting quantity of a modified form ("tagged-target" or "analogue") of the intended target molecule (analyte) in order to form an analogue-labelled antibody complex. A limiting quantity of analogue is applied to the mixture, to avoid the analogue out competing the naturally occurring analyte present in the sample for binding to the antibody. Thus, due to the configuration of the assay, the greater the amount of analyte present in the sample under investigation, a lesser amount of the analogue will be captured by the first binding moiety. The assay is thus considered a negative read immunoassay, since the detectable signal derived from the labelled antibody is inversely proportional to the concentration of analyte of interest present in the sample.

In a second phase the analogue-labelled antibody complexes are applied to a solid phase on which is immobilised a capture species specific for a unique tag on the analogue molecule in a reaction chamber. The solid phase is loaded with an excess of capture molecule to ensure efficient capture of any analogue-labelled antibody complexes present in solution. Following capture of analogue-labelled antibody complexes on the solid phase capture molecule, any unbound labelled antibodies (i.e. those which are complexed with the target analyte) are removed from the reaction chamber by a washing step, leaving only the analogue-labelled antibody complexes that have been captured on the solid phase. Thereafter, the presence of label is quantified using a suitable method, in accordance with the label that has been used.

The label on the antibody may be selected from an enzyme, a fluor, a radionuclide, a colloidal sol, a chromophore, luminescent compound. Depending on the enzyme selected the reaction product of the enzyme may either be determined optically (absorbance or fluorescence or visually) or electrochemically.

Preferred features of each aspect of the invention are as for each of the other aspects *mutatis mutandis.*

### Description of the Figures

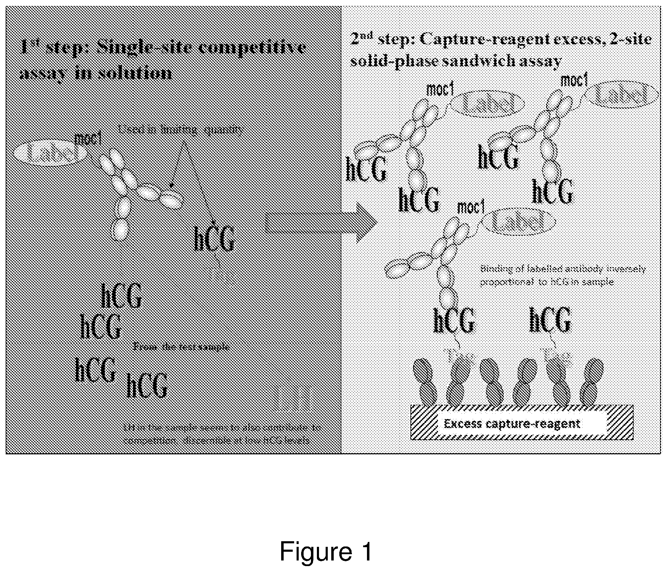

Figure 1 represents a schematic description of a hybrid competitive immunoassay according to an embodiment of the invention, and indicates possible cross reaction that might occur between the target analyte and another species present or potentially present in the sample. In an exemplary embodiment where the target analyte is hCG, the cross reacting species is LH.

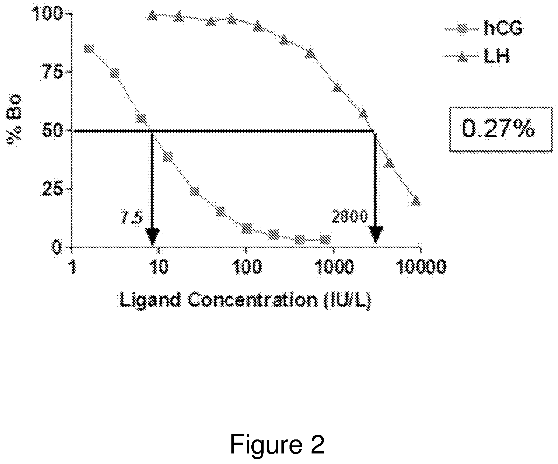

Figure 2 represents an inhibition curve, indicating the degree with which a selected monoclonal antibody for an hCG assay, moc1, that has been raised against the conserved β3-hCG loop region, cross reacts with LH. The data indicate cross reactivity with LH is 0.27% compared with hCG, based on the quantity of ligand needed to saturate 50% of the antibody.

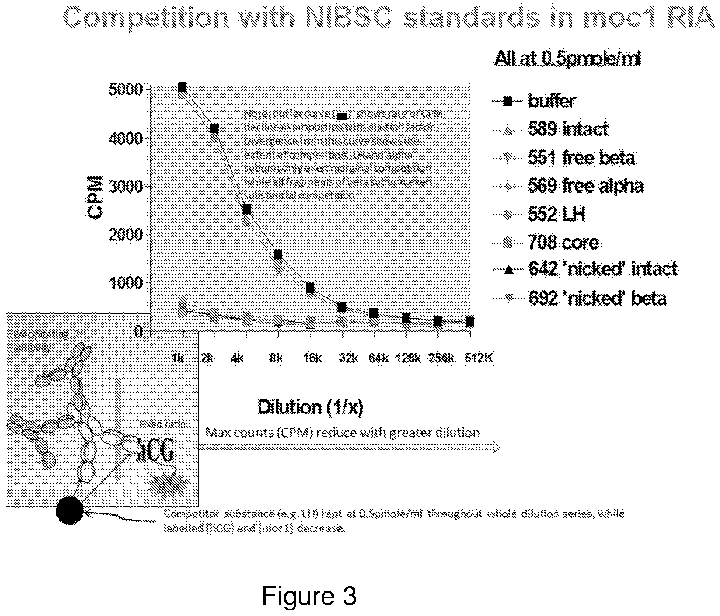

Figure 3 provides further characterising data that demonstrate the specificity of the monoclonal antibody, moc1, for binding to βhCG. The graph represents number of counts on the y-axis due to I125 radiolabelled moc1 that has bound tagged hCG, and the x-axis indicates the level of dilution from 1000^{th} to 512000^{th}. The data represent a series of curves that demonstrate the ability of the moc1 antibody to distinguish between βhCG and either αhCG or LH. The buffer control, 569 free alpha and LH all follow a similar profile, with high counts at low dilution, declining as sample is diluted. However, when the antibody and tagged hCG are incubated with either free βhCG, "nicked" βhCG, intact hCG, "nicked" hCG or "core" hCG (unlabelled target), the signals are significantly suppressed, even at low dilution, because the unlabelled target has successfully outcompeted the tagged-hCG for binding to the antibody.
Figure 4 represents a comparison of recombinant moc1 Fab₁ (monomer) and Fab₂ (dimer).
Figure 5 shows a comparison between the serum TAG assay and the RIA assay based on samples with low to high hCG concentrations. The x-axis displays the hCG concentration (IU/L) measured via the TAG assay. The y-axis displays the hCG concentration (IU/L) measured via the RIA assay.
Figure 6 shows a comparison between the serum TAG assay and the Immulite assay based on samples with low to high hCG concentrations. The x-axis displays the hCG concentration (IU/L) measured via the TAG assay. The y-axis displays the hCG concentration (IU/L) measured via the Immulite assay.
Figure 7 shows a comparison between the serum TAG assay and the RIA assay based on samples with mid to high hCG concentrations. The x-axis displays the hCG concentration (IU/L) measured via the TAG assay. The y-axis displays the hCG concentration (IU/L) measured via the RIA assay.
Figure 8 shows a comparison between the serum TAG assay and the Immulite assay based on samples with mid to high hCG concentrations. The x-axis displays the hCG concentration (IU/L) measured via the Immulite assay. The y-axis displays the hCG concentration (IU/L) measured via the TAG assay.

### Example 1 - Protocol for serum TAG assay

### Reagents

| | |
|---|---|
| Standard diluent | - Serasub (SS) |
| Sample pre-diluent | - Normal sheep serum (NSS) |
| moc1 | - 10ul stock (1:1000) in 4ml 3% BSA/1% NSS/PBS |
| TAG | - 10ul stock (1:1000) in 4ml 3% BSA/1% NSS/PBS |
| Microtitre plate | - pre-coated with anti TAG |

### Sample preparation

| | |
|---|---|
| 0-100 IU/L | - dilute 1:2 in SS. |
| 100-1000 | - dilute 1:5 in NSS then further in SS as required. |
| >1000 | - dilute 1:50 in NSS then further in SS as required |

### Assay

- Prepare standard curve by serial diluting top hCG standard (100 IU/L) six times in SS to generate 50, 25, 12.5, 6.25, 3.125 and 1.56 IU/L standards. Include SS alone to give a zero standard.
- Dilute serum samples as appropriate (detailed above).
- Using a multichannel pipette, add 35µl of moc1 solution to wells of the microtitre plate.
- Add 35µl of standards and samples.
- Using a multichannel pipette, add 35µl of TAG solution to all wells.
- Shake plate for 1 hour at room temperature.
- Remove the content of the wells by tipping and blotting onto tissue paper.
- Wash 6x with 0.1% Tween/PBS.
- Add 100µl femto substrate and read on BMG Fluostar luminometer.
- Calculate results using BMG Omega Mars data analysis software.

### Example 2 - Protocol for urine TAG assay

### Reagents

| | |
|---|---|
| Standard diluent | - Urisub (US) |
| Sample diluent | - 10% Normal sheep serum (NSS)/US |
| moc1 | - 10ul stock (1:1000) in 4ml 3% BSA/1% NSS/20%FCS/PBS |
| TAG | - 2.3ul stock (1:1000) in 4ml 3% BSA/1% NSS/PBS |
| Microtitre plate | - pre-coated with anti TAG |

### Sample preparation

| | |
|---|---|
| 0-100 IU/L | - Neat |
| >100 IU/L | - dilute in 10% NSS/US |

### Assay

- Prepare standard curve by serial diluting top hCG standard (100 IU/L) six times in US to generate 50, 25, 12.5, 6.25, 3.125 and 1.56 IU/L standards. Include SS alone to give a zero standard.
- Dilute urine samples as appropriate (detailed above).
- Using a multichannel pipette, add 35µl of moc1 solution to wells of the microtitre plate.
- Add 35µl of standards and samples.
- Using a multichannel pipette, add 35µl of TAG solution to all wells.
- Shake plate then incubate for 2 hours at 37°C.
- Remove the content of the wells by tipping and blotting onto tissue paper.
- Wash 6x with 0.1% Tween/PBS.
- Add 100µl femto substrate and read on BMG Fluostar luminometer (see settings data).
- Calculate results using BMG Omega Mars data analysis software (see analysis data).

### Example 3 - Comparison of moc1 Fab variants

Recombinant moc1 Fab₁ (monomer) and Fab₂ (dimer) were produced in *E. coli* by conventional methods known in the art by the skilled person. Each Fab variant (monomeric variant and dimeric variant) was purified and conjugated to horseradish peroxidase (HRP) using Lightning Link HRP kits (Innova Bioscience) according to the manufacturer's instructions. The molecular weight of each Fab variant was taken into account for the conjugation process so that the monomeric Fab₁ variant and dimeric Fab₂ variant were subjected to comparable levels of labelling with HRP.

Following labelling with HRP and initial activity testing, the labelled Fab variants were assessed in a competition assay using TAG and hCG in order to derive a standard curve following the general method provided in Example 1.

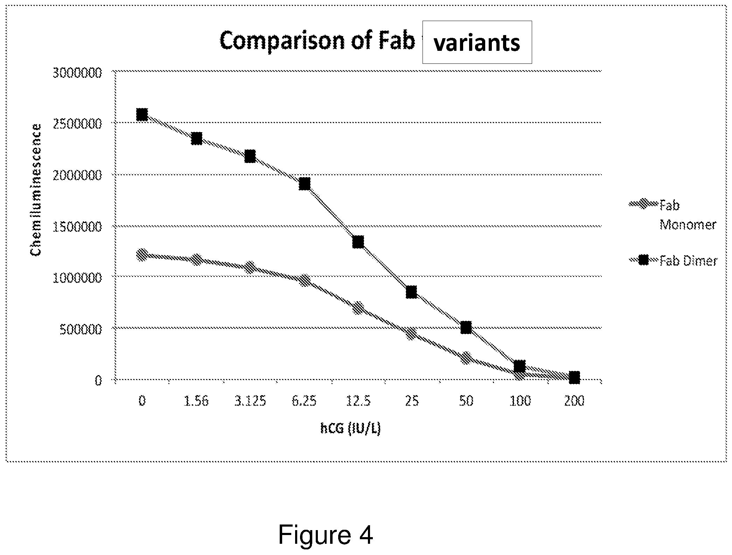

The results of the assays are given in Table 1 and Figure 4.

**Table 1. Averaged data showing assay output and % Signal relative to negative hCG standard**

| **hCG standard (IU/L)** | **Chemiluminescence (RLU)** | | % signal | |
|---|---|---|---|---|
| | **Monomer** | **Dimer** | **Monomer** | **Dimer** |
| 0 | 1202503 | 2572679 | 100 | 100 |
| 1.56 | 1165822 | 2346433 | 97 | 91 |
| 3.125 | 1080330 | 2162202 | 90 | 84 |
| 6.25 | 961748 | 1900875 | 80 | 73.9 |
| 12.5 | 687755 | 1340029 | 57 | 52 |
| 25 | 440407 | 848495 | 36.6 | 33 |
| 50 | 197693 | 504683 | 16.4 | 19.6 |
| 100 | 49539 | 130589 | 4 | 5 |
| 200 | 11774 | 23302 | 1 | 0.9 |

Table 1 and Figure 4 demonstrate that the Fab₂ dimer variant has a significant, approximately twofold, increase in chemiluminescent output relative to the Fab₁ monomer variant under comparable assay conditions. In addition, the Fab₂ dimer variant has a greater sensitivity to hCG concentration in the sample over the hCG concentration range 1.56-25 IU/L relative to the Fab₁ monomer variant as indicated by the more acute decrease in % signal across this range (see Figure 4).

### Example 4 - Clinical studies comparing the TAG assay with Immulite and RIA assays

Studies were performed to compare the ability of the TAG assay employing the Fab₂ dimer variant as described herein and known assays, the Immulite assay and RIA assay, to detect hCG in clinical samples.

Clinical serum samples were analysed for the presence and concentration of hCG by each assay method. The TAG assay was performed as described in Example 1. Immulite assays (Siemens) were performed according to the manufacturer's instructions. RIA assays were performed according to protocol instructions provided by the National Health Service, UK. In short, the assay uses a purified polyclonal antibody and iodinated hCG wherein sample is diluted and mixed with antibody and labelled hCG for 24 hours prior to measurement.

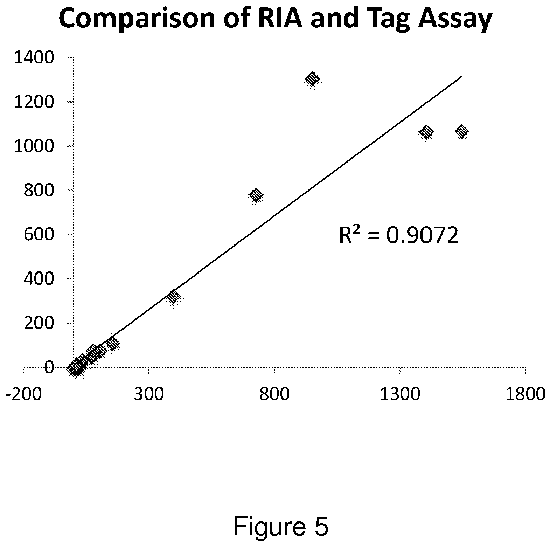

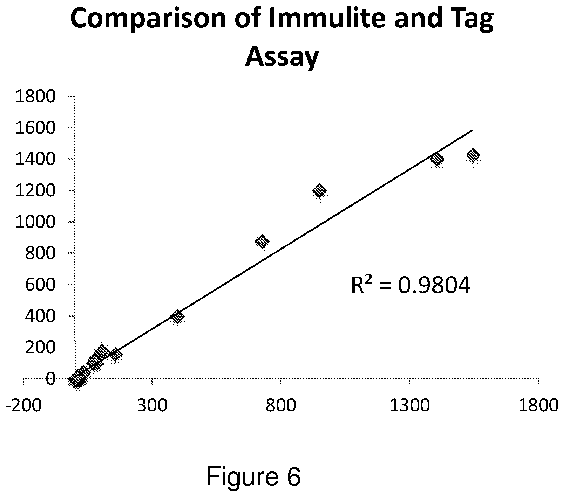

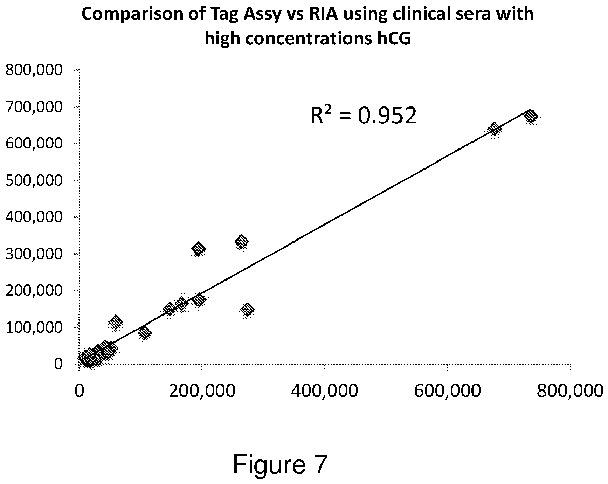

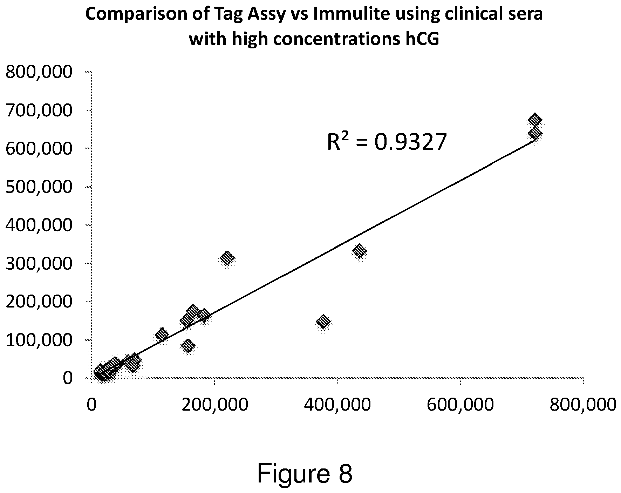

The results of the assays is given in Tables 2 and 3 and Figures 5-8.

**Table 2. hCG detection in clinical serum samples via the TAG assay, Immulite assay or RIA assay method whereby the hCG concentration range across the samples was from low to high hCG concentrations.**

| | **hCG concentration (IU/L)** | | |
|---|---|---|---|
| **Sample** | **TAG** | **RIA** | **Immulite** |
| 1 | **17** | 13 | 16 |
| 2 | **5** | 4 | 5 |
| 3 | **70** | 46 | 100 |
| 4 | **18** | 5 | 7 |
| 5 | **7** | 5 | 8 |
| 6 | **104** | 75 | 176 |
| 7 | **26** | 14 | 33 |
| 8 | **397** | 321 | 398 |
| 9 | **14** | 10 | 11 |
| 10 | **1163** | 1148 | 1279 |
| 11 | **82** | 70 | 95 |
| 12 | **76** | 75 | 120 |
| 13 | **1391** | 1336 | 1834 |
| 14 | **726** | 779 | 875 |
| 15 | **15** | 5 | 5 |
| | | | |
| 16 | **949** | 1305 | 1200 |
| | | | |
| 17 | **23** | 11 | 28 |
| | | | |
| 18 | **34040** | 49380 | 58146 |
| | | | |
| | | | |
| 19 | **96546** | 122920 | 177465 |
| | | | |
| 20 | **154** | 108 | 156 |
| 21 | **14** | 12 | 13 |
| 22 | **175265** | 194800 | 164721 |
| 23 | **24615** | 22330 | 25561 |
| 24 | **1545** | 1067 | 1425 |
| 25 | **3** | <4 | <1 |
| 26 | **2** | <4 | <1 |
| 27 | **12** | 9 | 13 |
| 28 | **1405** | 1063 | 1402 |
| 29 | **8** | 9 | 11 |
| 30 | **1** | <4 | <1 |
| 31 | **33** | 32 | 39 |
| 32 | **2** | <4 | <1 |
| 33 | **9** | 6 | 10 |
| 34 | **10** | 9 | 13 |

**Table 3. hCG detection in clinical serum samples via the TAG assay, Immulite assay or RIA assay method whereby the hCG concentration range across the samples was from mid to high hCG concentrations.**

| **hCG concentration (IU/L)** | | |
|---|---|---|
| Immulite | RIA | TAG |
| 164721 | 194800 | 175265 |
| 25561 | 22330 | 24615 |
| 21884 | 17700 | 19028 |
| 721139 | 676000 | 640363 |
| 21884 | 17290 | 12977 |
| 721139 | 735400 | 675028 |
| 182140 | 166750 | 164552 |
| 39602 | 30430 | 36581 |
| 26551 | 19230 | 23276 |
| 58258 | 51900 | 44120 |
| 36871 | 41600 | 38110 |
| 25546 | 35000 | 23997 |
| 17112 | 13350 | 12132 |
| 154163 | 147200 | 150430 |
| 17494 | 17200 | 14079 |
| 14202 | 10210 | 14459 |
| 14230 | 9700 | 19655 |
| 220085 | 193900 | 314760 |
| 69034 | 41860 | 48667 |
| 435384 | 264200 | 333333 |
| 19546 | 19670 | 15863 |
| 22642 | 14285 | 21467 |
| 27591 | 25800 | 14364 |
| 156336 | 106600 | 86333 |
| 376837 | 273500 | 148267 |
| 28086 | 18550 | 15583 |
| 66289 | 46560 | 33289 |
| 114379 | 59160 | 114315 |
| 28862 | 16770 | 26918 |

Figures 5-8 demonstrate that the detection and measurement of hCG concentration in clinical serum samples via TAG assay correlates strongly with the known Immulite and RIA assays with R² values greater than 0.9 in all cases. Some variation between assays methods exists due to differences in antibody recognition of the target epitopes on hCG, however all assays can accurately detect hCG in clinical sera.

Furthermore, further clinical studies have demonstrated that the key benefit of the TAG assay is its ability to report specifically with good sensitivity. It has been shown to detect very low levels of hCG and detect increases in patient samples before the known RIA and Immulite assays were able to do so which is invaluable in monitoring patients undergoing therapy, particularly when the levels of hCG are very low. In addition to the superior sensitivity of the TAG assay over known assay methods, the TAG assay is laboratory based and can be completed in a much shorter period of time (4 hours) than the known assays using simple laboratory facilities and equipment.

## Claims

1. A method of determining the presence of an analyte present in a liquid sample, the method comprising:
a) contacting the liquid sample with a predetermined amount of an analogue of the analyte and a first binding moiety, wherein:
the analogue comprises an amino acid sequence which has been recombinantly expressed or synthesized *in vitro* to include a binding region composed of amino acids that can be bound by a second binding moiety, such that the analogue incorporates only one of said binding region, wherein the binding region is not present in the analyte or is present in the analyte but not accessible to the second binding moiety; and
the first binding moiety is capable of binding each of the analyte and the analogue independently, such that a mixture is formed comprising an analogue-first binding moiety complex, or, when analyte is present in the sample, either (i) an analogue-first binding moiety complex and an analyte-first binding moiety complex, or (ii) an analyte-first binding moiety complex and no analogue-first binding moiety complex;
b) contacting the mixture with the second binding moiety, wherein the second binding moiety is an antibody capable of binding the analogue-first binding moiety complex but not the analyte-first binding moiety complex, and is immobilised or immobilisable on a solid phase;
c) determining the level of a signal indicative of the presence of the analogue-first binding moiety complex bound to the second binding moiety;
wherein if the level of the signal determined in step c) is lower than the level of a maximum signal determined when no analyte is present, then analyte is present in the sample.

2. The method of claim 1 wherein the first binding moiety is capable of binding each of the analyte and the analogue independently but not both the analogue and analyte simultaneously.

3. The method of claim 1 or claim 2, wherein the sample is contacted with the first binding moiety prior to being contacted with the analogue.

4. The method of any one of claims 1 to 3, further comprising a step of determining the amount or concentration of analyte present in the sample by comparing the level of signal determined in step c) with calibration data.

5. The method of any preceding claim, wherein the analyte is hCG or a fragment or portion thereof.

6. The method of any preceding claim, wherein the signal is derived from a label that is either directly or indirectly associated with the first-binding moiety.

7. The method of claim 6, wherein the label is selected from the group consisting of an enzyme, a fluorophore, a radionuclide, a colloidal sol, a chromophore and a luminescent compound.

8. The method of any preceding claim, wherein the analogue includes an amino acid sequence that is identical to either the full analyte sequence or a portion of the analyte sequence.

9. The method of any preceding claim, wherein the first binding moiety is an antibody.

10. The method of any preceding claim, wherein the first binding moiety is a Fab or F(ab)₂.

11. The method of any preceding claim, wherein the first binding moiety binds specifically to hCG.

12. The method of any one of claims 1 to 9, wherein the first binding moiety is an hCG β3-loop-specific monoclonal antibody.

13. The method of any preceding claim, wherein the sample is derived from or consists of a physiological source including blood, serum, plasma, interstitial liquid, saliva, sputum, ocular lens liquid, sweat, urine, milk, ascites liquid, mucous, synovial liquid, peritoneal liquid, transdermal exudates, pharyngeal exudates, bronchoalveolar lavage, tracheal aspirations, cerebrospinal liquid, semen, cervical mucus, vaginal or urethral secretions or amniotic liquid.

14. A test kit for detecting an analyte of interest comprising
i) an analyte analogue;
ii) either (a) a labelled first binding moiety or (b) an unlabelled first binding moiety and a labelled third binding moiety; and
iii) an immobilisable second binding moiety
wherein:
the analogue comprises an amino acid sequence which has been recombinantly expressed or synthesized *in vitro* to include a binding region composed of amino acids that can be bound by the second binding moiety, such that the analogue incorporates only one of said binding region, wherein the binding region is not present in the analyte or is present in the analyte but not accessible to the second binding moiety; and
the first binding moiety is capable of binding each of the analogue and the analyte of interest independently, the second binding moiety is an antibody capable of binding a complex of the analogue and the first binding moiety but is not capable of binding the analyte or a complex of the first binding moiety and the analyte, and the third binding moiety is capable of binding the first binding moiety or the analogue-first binding moiety complex.

15. The test kit of claim 14 wherein the first binding moiety is capable of binding each of the analogue and the analyte of interest independently but not both the analogue and analyte simultaneously.

## Patentansprüche

1. Verfahren zur Bestimmung der Anwesenheit eines Analyten in einer flüssigen Probe, wobei das Verfahren umfasst:
a) Inkontaktbringen der flüssigen Probe mit einer vorbestimmten Menge eines Analogons des Analyten und einer ersten Bindungseinheit, worin:
das Analogon eine Aminosäuresequenz umfasst, welche rekombinant exprimiert oder in vitro synthetisiert wurde, so dass sie eine Bindungsregion einschließt, welche aus Aminosäuren besteht, die durch eine zweite Bindungseinheit gebunden werden können, wobei das Analogon nur eine derartige Bindungsregion inkorporiert, worin die Bindungsregion in dem Analyten nicht vorhanden ist oder in dem Analyten vorhanden, jedoch nicht für die zweite Bindungseinheit zugänglich ist; und
die erste Bindungseinheit fähig ist, sowohl den Analyten als auch das Analogon unabhängig voneinander zu binden, so dass eine Mischung gebildet wird, welche einen Komplex aus Analogon und erster Bindungseinheit umfasst oder, wenn Analyt in der Probe vorhanden ist, entweder (i) einen Komplex aus Analogon und erster Bindungseinheit und einen Komplex aus Analyt und erster Bindungseinheit, oder (ii) einen Komplex aus Analyt und erster Bindungseinheit und keinen Komplex aus Analogon und erster Bindungseinheit umfasst;
b) Inkontaktbringen der Mischung mit der zweiten Bindungseinheit, worin die zweite Bindungseinheit ein Antikörper ist, der fähig ist, den Komplex aus Analogon und erster Bindungseinheit, jedoch nicht den Komplex aus Analyt und erster Bindungseinheit zu binden, und der immobilisiert oder immobilisierbar auf einer festen Phase ist;
c) Bestimmen der Stärke des Signals, welches die Gegenwart des an die zweite Bindungseinheit gebundenen Komplexes aus Analogon und erster Bindungseinheit anzeigt;
wobei Analyt in dem Fall in der Probe vorhanden ist, in dem die Stärke des in Schritt c) bestimmten Signals niedriger ist als eine maximale Signalstärke, die bestimmt wurde, wenn kein Analyt vorhanden ist.

2. Verfahren nach Anspruch 1, worin die erste Bindungseinheit fähig ist, sowohl den Analyten als auch das Analogon unabhängig voneinander zu binden, jedoch nicht Analogon und Analyt gleichzeitig.

3. Verfahren nach Anspruch 1 oder Anspruch 2, worin die Probe mit der ersten Bindungseinheit in Kontakt gebracht wird, bevor sie mit dem Analogon in Kontakt gebracht wird.

4. Verfahren nach einem der Ansprüche 1 bis 3, weiter umfassend einen Schritt der Bestimmung der Menge oder Konzentration von in der Probe vorhandenem Analyt durch Vergleichen der in Schritt c) bestimmten Signalstärke mit Kalibrierungsdaten.

5. Verfahren nach einem der vorhergehenden Ansprüche, worin der Analyt hCG oder ein Fragment oder Teil hiervon ist.

6. Verfahren nach einem der vorhergehenden Ansprüche, worin das Signal aus einer Markierung stammt, die entweder direkt oder indirekt mit der ersten Bindungseinheit assoziiert ist.

7. Verfahren nach Anspruch 6, worin die Markierung ausgewählt ist aus der Gruppe bestehend aus einem Enzym, einem Fluorophor, einem Radionuklid, einem kolloidalen Sol, einem Chromophor und einer luminiszierenden Verbindung.

8. Verfahren nach einem der vorhergehenden Ansprüche, worin das Analogon eine Aminosäuresequenz einschließt, die identisch zur gesamten Analytsequenz oder zu einem Teil der Analytsequenz ist.

9. Verfahren nach einem der vorhergehenden Ansprüche, worin die erste Bindungseinheit ein Antikörper ist.

10. Verfahren nach einem der vorhergehenden Ansprüche, worin die erste Bindungseinheit ein Fab oder F(ab)₂ ist.

11. Verfahren nach einem der vorhergehenden Ansprüche, worin die erste Bindungseinheit spezifisch an hCG bindet.

12. Verfahren nach einem der Ansprüche 1 bis 9, worin die erste Bindungseinheit ein hCG β3-loop-spezifischer monoklonaler Antikörper ist.

13. Verfahren nach einem der vorhergehenden Ansprüche, worin die Probe erhalten ist aus oder besteht aus einer physiologischen Quelle einschließlich Blut, Serum, Plasma, interstitieller Flüssigkeit, Speichel, Sputum, Augenlinsenflüssigkeit, Schweiß, Urin, Milch, Ascitesflüssigkeit, Schleim, synovialer Flüssigkeit, Peritonealflüssigkeit, transdermalen Exsudaten, pharyngalen Exsudaten, Bronchoalveolarlavage, Trachealaspirationen, Cerebrospinalflüssigkeit, Sperma, Cervicalschleim, Vaginal- oder Harnröhrensekretionen oder Fruchtwasser.

14. Test-Kit zur Bestimmung eines interessierenden Analyten umfassend
i) ein Analytanalogon;
ii) entweder (a) eine markierte erste Bindungseinheit oder (b) eine nicht markierte erste Bindungseinheit und eine markierte dritte Bindungseinheit; und
iii) eine immobilisierbare zweite Bindungseinheit
wobei:
das Analogon eine Aminosäuresequenz umfasst, welche rekombinant exprimiert oder in vitro synthetisiert wurde, so dass sie eine Bindungsregion einschließt, welche aus Aminosäuren aufgebaut ist, die durch eine zweite Bindungseinheit gebunden werden können, wobei das Analogon nur eine derartige Bindungsregion inkorporiert, worin die Bindungsregion in dem Analyten nicht vorhanden ist, oder in dem Analyten vorhanden, jedoch für die zweite Bindungseinheit nicht zugänglich ist; und
die erste Bindungseinheit fähig ist, sowohl das Analogon als auch den interessierenden Analyten unabhängig voneinander zu binden, die zweite Bindungseinheit ein Antikörper ist, welcher einen Komplex des Analogons und der ersten Bindungseinheit binden kann, jedoch nicht fähig ist, den Analyten oder einen Komplex aus der ersten Bindungseinheit und dem Analyten zu binden, und die dritte Bindungseinheit fähig ist, die erste Bindungseinheit oder den Komplex aus dem Analogon und der ersten Bindungseinheit zu binden.

15. Test-Kit nach Anspruch 14, worin die erste Bindungseinheit fähig ist, sowohl das Analogon und den interessierenden Analyten unabhängig voneinander zu binden, jedoch nicht das Analogon und den Analyten gleichzeitig.

## Revendications

1. Procédé de détermination de la présence d'un analyte présent dans un échantillon liquide, le procédé comprenant :
a) la mise en contact de l'échantillon liquide avec une quantité prédéterminée d'un analogue de l'analyte et un premier fragment de liaison, où :
l'analogue comprend une séquence d'acides aminés qui a été exprimée de manière recombinante ou synthétisée *in vitro* pour inclure une région de liaison composée d'acides aminés qui peuvent être liés par un deuxième fragment de liaison, de sorte que l'analogue incorpore une seule de ladite région de liaison, où la région de liaison n'est pas présente dans l'analyte ou est présente dans l'analyte mais non accessible au deuxième fragment de liaison ; et
le premier fragment de liaison est capable de se lier à chacun de l'analyte et de l'analogue de manière indépendante, de sorte à former un mélange comprenant un complexe analogue-premier fragment de liaison, ou, lorsqu'un analyte est présent dans l'échantillon, soit (i) un complexe analogue-premier fragment de liaison et un complexe analyte-premier fragment de liaison, soit (ii) un complexe analyte-premier fragment de liaison et aucun complexe analogue-premier fragment de liaison ;
b) la mise en contact du mélange avec le deuxième fragment de liaison, où le deuxième fragment de liaison est un anticorps capable de se lier au complexe analogue-premier fragment de liaison mais pas au complexe analyte-premier fragment de liaison, et est immobilisé ou peut être immobilisé sur une phase solide ;
c) la détermination du niveau d'un signal indicatif de la présence du complexe analogue-premier fragment de liaison lié au deuxième fragment de liaison ;
dans lequel si le niveau du signal déterminé à l'étape c) est plus faible que le niveau d'un signal maximum déterminé lorsqu'aucun analyte n'est présent, alors l'analyte est présent dans l'échantillon.

2. Procédé selon la revendication 1, dans lequel le premier fragment de liaison est capable de se lier à chacun de l'analyte et de l'analogue de manière indépendante mais pas à l'analogue et à l'analyte de manière simultanée.

3. Procédé selon la revendication 1 ou la revendication 2, dans lequel l'échantillon est mis en contact avec le premier fragment de liaison avant d'être mis en contact avec l'analogue.

4. Procédé selon l'une quelconque des revendications 1 à 3, comprenant en outre une étape consistant à déterminer la quantité ou la concentration d'analyte présente dans l'échantillon en comparant le niveau du signal déterminé à l'étape c) avec des données d'étalonnage.

5. Procédé selon une quelconque revendication précédente, dans lequel l'analyte est la hCG ou un fragment ou une partie de celle-ci.

6. Procédé selon une quelconque revendication précédente, dans lequel le signal est dérivé d'un marqueur qui est directement ou indirectement associé au premier fragment de liaison.

7. Procédé selon la revendication 6, dans lequel le marqueur est sélectionné dans le groupe consistant en une enzyme, un fluorophore, un radionucléide, un sol colloïdal, un chromophore et un composé luminescent.

8. Procédé selon une quelconque revendication précédente, dans lequel l'analogue comprend une séquence d'acides aminés qui est identique à l'intégralité de la séquence de l'analyte ou à une partie de la séquence de l'analyte.

9. Procédé selon une quelconque revendication précédente, dans lequel le premier fragment de liaison est un anticorps.

10. Procédé selon une quelconque revendication précédente, dans lequel le premier fragment de liaison est un Fab ou F(ab)₂.

11. Procédé selon une quelconque revendication précédente, dans lequel le premier fragment de liaison se lie spécifiquement à la hCG.

12. Procédé selon l'une quelconque des revendications 1 à 9, dans lequel le premier fragment de liaison est un anticorps monoclonal spécifique pour la boucle β3 de la hCG.

13. Procédé selon une quelconque revendication précédente, dans lequel l'échantillon est dérivé de ou consiste en une source physiologique comprenant du sang, du sérum, du plasma, du liquide interstitiel, de la salive, des expectorations, des larmes, de la sueur, de l'urine, du lait, du liquide d'ascites, du mucus, du liquide synovial, du liquide péritonéal, des exsudats transdermiques, des exsudats pharyngés, un lavage broncho-alvéolaire, des aspirations trachéales, du liquide céphalorachidien, du sperme, de la glaire cervicale, des sécrétions vaginales ou urétrales ou du liquide amniotique.

14. Kit de test pour détecter un analyte d'intérêt comprenant
i) un analogue de l'analyte ;
ii) soit (a) un premier fragment de liaison marqué ou (b) un premier fragment de liaison non marqué et un troisième fragment de liaison marqué ; et
iii) un deuxième fragment de liaison pouvant être immobilisé
dans lequel :
l'analogue comprend une séquence d'acides aminés qui a été exprimée de manière recombinante ou synthétisée *in vitro* pour inclure une région de liaison composée d'acides aminés qui peuvent être liés par le deuxième fragment de liaison, de sorte que l'analogue incorpore une seule de ladite région de liaison, où la région de liaison n'est pas présente dans l'analyte ou est présente dans l'analyte mais non accessible au deuxième fragment de liaison ; et
le premier fragment de liaison est capable de se lier à chacun de l'analogue et de l'analyte d'intérêt de manière indépendante, le deuxième fragment de liaison est un anticorps capable de se lier à un complexe entre l'analogue et le premier fragment de liaison mais n'est pas capable de se lier à l'analyte ou à un complexe entre le premier fragment de liaison et l'analyte, et le troisième fragment de liaison est capable de se lier au premier fragment de liaison ou au complexe analogue-premier fragment de liaison.

15. Kit de test selon la revendication 14, dans lequel le premier fragment de liaison est capable de se lier à chacun de l'analogue et de l'analyte d'intérêt de manière indépendante mais pas à l'analogue et à l'analyte de manière simultanée.
